# EUROPEAN PATENT SPECIFICATION

(11) **EP 3 068 355 B1**
(45) Date of publication and mention of the grant of the patent: **11.10.2017**
(21) Application number: 14833607.6
(22) Date of filing: 11.11.2014
(51) Int. Cl.: A61F 9/008

(54) **OPHTHALMIC LASER DEVICE**
AUGENLASERVORRICHTUNG
DISPOSITIF DE LASER OPHTALMOLOGIQUE

(30) Priority: 11.11.2013 SI 201300379
(43) Date of publication of application: 21.09.2016
(73) Proprietor: Optotek d.o.o., 1000 Ljubljana (SI)
(72) Inventor: VRECKO, Andrej, 1000 Ljubljana (SI); VEDLIN, Boris, 1000 Ljubljana (SI); JAKISA, Jasa, 1000 Ljubljana (SI); GREGORCIC, Peter, 1000 Ljubljana (SI)
(74) Representative: Flak, Antonija
(86) International application number: PCT/SI2014/000064
(87) International publication number: WO 2015/069197

(56) References cited:
- WO-A1-01/91661
- RU-A- 2009 137 641
- US-A1- 2012 259 321

## Description

### Field of the invention

The present invention is a device for performing therapeutic procedure of selective laser trabeculoplasty for treatment of open-angle glaucoma which allows autonomous adjustment of energy values of the device and thus more effective operation procedure.

The present invention belongs to the field of devices for performing selective laser trabeculoplasty, more precisely to the field of systems for adjustment of laser pulse energy with the help of automatic detection of trabecular bubble formation on the patient's eye in the therapeutic device itself, which allows setting of optimal laser pulse energy during procedure. Devices wherein the bubble formation is measured and the laser settings adapted accordingly are discussed for example in WO 01/91661.

### Background and the technical problem

The technical problem, which is solved by the present invention, is how to allow adjustment of laser pulse energy, with the help of automatic detection of trabecular bubble formation on the patient's eye, in the therapeutic device for selective laser trabeculoplasty itself, which would enable adjusting of the optimal energy of laser pulses during procedure. Automatic adjusting of laser pulse energy would allow autonomous functioning of the device, which would also increase the ability to perform operation procedures closer to the threshold for trabecular bubble formation, consequently leading to higher operation reproducibility. The problem of bubble detection, including their size, number, speed and direction, which are formed at energies that exceed the threshold for trabecular bubble formation during glaucoma operation with SLT method, and consequently regulation and choice of energy values of laser pulses, should be solved in a relatively easy, inexpensive and technically reliable way.

Glaucoma is a common eye disease, in which the optic nerve is injured on the site, where it exits the eye. Due to these injuries sight irreversibly deteriorates. The frequency of glaucoma increases with age; at the age of 80 more than 4% of population suffer from glaucoma. The optic nerve defect in glaucoma is most commonly associated with ocular hypertension, which occurs due to hampered outflow of aqueous humour through the trabecular tissue. If the eye pressure is too high, the optic nerve is mechanically damaged, which consequently leads to nerve fibre necrosis. Costs for medical treatment with medicaments are very high; therefore a need for different solutions has emerged. The treatment technique with lasers is known as laser trabeculoplasty. It was discovered by Latina and is described in patent US5549596, owned by The General Hospital Corporation. Latina described the use of pulsed, frequency-doubled Nd:YAG laser for laser trabeculoplasty.

Selective laser trabeculoplasty (hereinafter SLT) is an improvement of an older technique called argon laser trabeculoplasty (ALT). Argon laser trabeculoplasty causes permanent coagulation damage (due to thermal effects) and can therefore be used only once in each eye. The SLT method, on the other hand, can be used more than once. This method does not cause coagulation of trabecular tissue, while selective absorption in melanin stimulates and facilitates drainage of aqueous humour. Due to selective mode of action when illuminating trabecular tissue, there are no visual changes in the tissue after application of a laser pulse, however, formation of trabecular bubbles can be observed, when the energy threshold for their formation is exceeded. Hence, operation protocol is closely linked with this energy threshold as will be described in more detail below.

Operation protocol of selective laser trabeculoplasty, when using existing commercially available solutions, is performed as follows. The doctor starts with the operation at low laser energy (typically 0.8 mJ) with pulses, which are shorter than 5 ns and have a wavelength of 532 nm. Then, the energy is increased until the doctor visually recognizes formation of trabecular bubbles through a binocular. When bubbles are detected, the operating doctor lowers laser energy just below the threshold for formation of trabecular bubbles. In this way the doctor subjectively regulates laser pulse energy during operation.

### State of the art

Known devices require that the operating doctor visually and subjectively recognizes formation of trabecular bubbles and on the basis of this subjective judgment adjusts the laser pulse energy on his own.

Patent EP1540403 discloses a laser system, which combines functions of two devices, a photodisruptor and a laser device for selective laser trabeculoplasty, in one device. This solution does not allow automatic detection of trabecular bubble formation and adjustment of laser pulse energy during operation.

Patent EP1377243 discloses an optical laser system, which allows execution of retina photocoagulation and laser trabeculoplasty, but does not enable control and automatic regulation of laser pulse energy during operation.

Patent S122509 discloses an optical system for selective laser trabeculoplasty, which ensures a homogenous density of energy and a sharp edge of a laser pulse point on the target. Also this solution does not solve the problem of regulation and selection of energy values of laser pulses during operation.

The present invention differs from known solutions in that it solves the problem of adjusting laser parameters in selective laser trabeculoplasty with the help of automatic detection of trabecular bubble formation on the patient's eye. This solution allows automatic adjustment of laser pulse energy and at the same time unburdens the doctor, who can devote more attention to the operation, which is not the case in the above mentioned known solutions. In addition, the present invention allows higher reproducibility of the whole procedure, as autonomous functioning of the device enables execution of procedures closer to the threshold for trabecular bubble formation, thereby making it more reproducible.

### Description of the invention

The essence of the ophthalmic laser device according to the invention is that the device includes an imaging system, which serves as the basis for automatic detection of trabecular bubbles during selective laser trabeculoplasty operation, and a processor system, which together with integrated algorithms analyses images, recognizes formation of trabecular bubbles during operation and triggers automatic regulation of laser pulse energy value. The invention is defined in the claims, other embodiments being merely exemplary. For a further understanding of the present invention, reference should be made to the following detailed disclosure based on an embodiment of ophthalmic laser device for selective laser trabeculoplasty, taken in conjunction with the accompanying figures, which show:

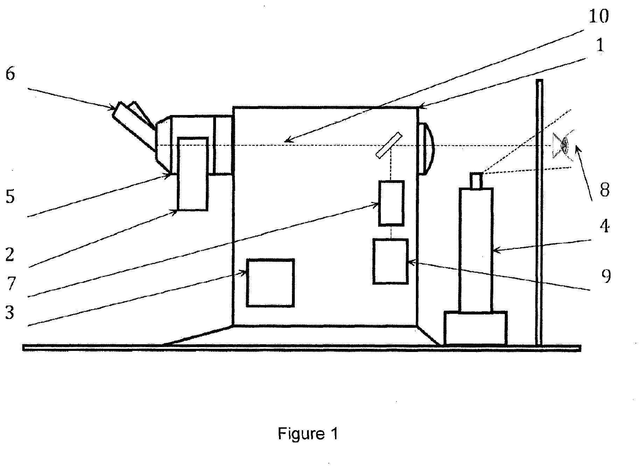

Figure 1 - Schematic drawing of the ophthalmic laser device
Figure 2 - Block diagram of the feedback loop
Figure 3 - Algorithm for autonomous adjustment of laser pulse energy
Figure 4 - Image of air bubbles
Ophthalmic laser device for performing selective laser trabeculoplasty functions in such a way that after activation of laser pulse in a beam splitter 5 a part of image from optical axis 10 of a biomicroscope is directed into an imaging system 2, which forwards taken images of patient's eye 8 to a processor system 3. The processor system 3 in dependence to received images from the imaging system 2 and an algorithm 11 for recognition of images on the basis of a predefined algorithm 12 controls a variable attenuation filter 7, which decreases or increases laser pulse energy.

The ophthalmic laser device according to the present invention allows, regardless of the used elements or algorithm types, detection of air bubbles, their size, number, speed and direction, which are formed at energies that exceed the threshold for trabecular bubble formation during operation of glaucoma with SLT method, on the basis of which the device warns the doctor or in autonomous mode reacts with an appropriate adjustment of variable attenuation filter settings. Autonomy, to which the device may itself decide to increase or decrease the selected energy, should be limited to few steps of increase or decrease of energy value. Typically, a step of energy increase or decrease is 0.1 mJ.

Ophthalmic laser device with automatic detection of trabecular bubbles in patient's eye 8 has an integrated imaging system 2 in the device 1 for selective laser trabeculoplasty itself, the system 2 being integrated via the beam splitter 5 before binocular 6. In the device 1 for selective laser trabeculoplasty the processor system 3 is included, which together with the algorithm 11 processes images from system 2 and based on the predefined algorithm 12 for adjustment of laser pulses energy controls the variable attenuation filter 7. A slit lamp 4, which is a part of the device 1, functions as an illumination device, which illuminates the trabecular tissue during procedure with SLT so that it allows the doctor 13 sufficient tissue visibility and at the same time sufficient visibility of pilot beam, with which the point of laser pulse application is selected. When taking images the important key is sufficient illumination, therefore the slit lamp 4 of the described device has the ability of short-term functioning or emitting pulses with typical durations of 100 ms at increased illumination, respectively. The interval of increased illumination is triggered together with the laser pulse and enables the imaging system 2 to capture high-quality sequential shots, which serve as the basis for further processing, with a typical capture of five consecutive images. In this way, additional illumination is not distracting nor for the patient nor for the doctor. The imaging system 2 may be any device whose purpose is to take image via an optical sensor (commercial / industrial cameras, digital cameras, SLR cameras, etc.).

The method of functioning of the whole device is as follows: in accordance with classic operative protocol trabecular bubbles occur at the point of too high laser pulse energy. The imaging system 2 takes images during operation, which may contain trabecular bubbles, and forwards it to the algorithm 11 for recognition of trabecular bubble formation. Based on received images the algorithm 11 obtains information about existence, size, speed and direction of bubbles and forwards this information to the algorithm 12 for adjustment of laser pulse energy. The algorithm 12 then sends a signal to the variable attenuation filter 7, which decreases the laser pulse energy to an appropriate level based on the size and/or number of bubbles, or increases the laser pulse energy in case trabecular bubbles have not been formed, respectively. Image processing and variable attenuation filter 7 setting is executed in less than 0.33 s, which allows autonomous functioning of the device at activation with highest frequency of 3 Hz. It is important to point out that the autonomous function of the device relates only to selection of laser pulse energy and not to activation of laser source 9, which is performed by the doctor 13. In case autonomous function of the device has been switched off, the laser pulse energy can be also adjusted by the doctor 13 based on his own judgment or based on information received from the algorithm 12 for adjustment of laser pulse energy.

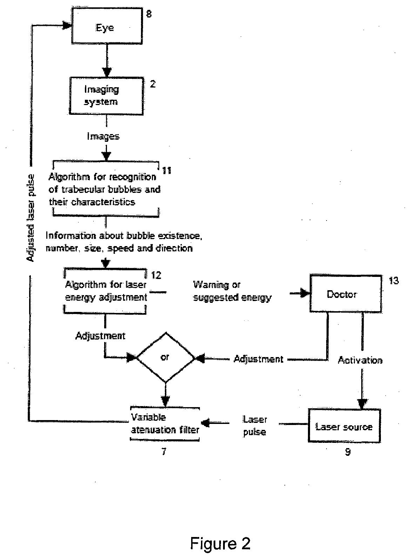

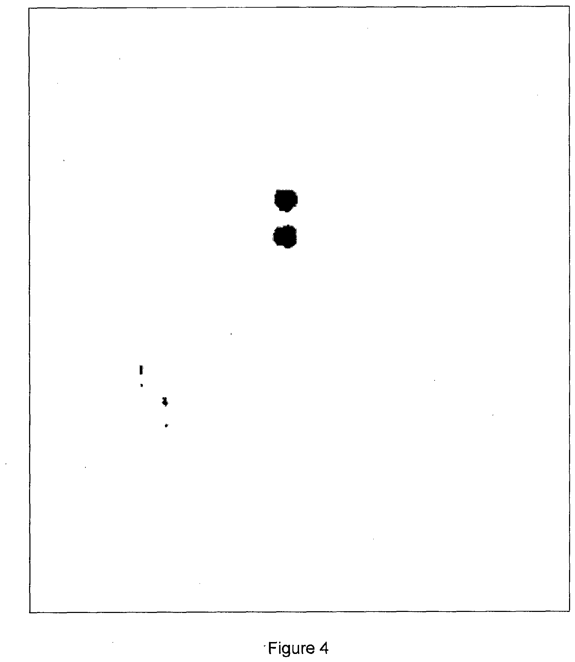

A block diagram representing the method of functioning of the device is shown in figure 2. After a performed laser pulse recognition of bubble formation is executed with the algorithm 11 for recognition of trabecular bubbles and their characteristics, which takes images from the imaging system 2 and then from a sequence of images or from one image, in case of stationary bubbles, selects shapes similar to bubbles, if they exist, as shown in figure 4. The algorithm 11 then proceeds with image processing and gains data from obtained images about bubble number, size, speed and direction of movement. Speed, shape and size are three of the most important criteria about existence of trabecular bubbles. The output parameter of algorithm 11 is information about existence of bubbles, their size and number. An example of how algorithm 11 works is that algorithm 11 searches for differences in three consecutive images, which are first transformed into 1-bit, black and white images. Algorithm 11 adds up the differences of 1-bit images and gains information about objects, which have moved. Then, the algorithm 11 collects at least two 1-bit images of movements (figure 4) and on the basis of pre-set criteria determines whether images contain bubbles or not. Pre-set criteria are shape, speed and size of bubble (object). In case bubble exists and has been recognized, the algorithm 11 classifies bubbles as small or big depending on their size and speed. Algorithm 11 for bubble recognition then forwards information about bubble existence, their number and size to the algorithm 12 for adjustment of laser pulse energy, which on the basis of predefined values together with the variable attenuation filter 7 appropriately decreases or increases the laser pulse energy. Typical change of energy value is 0.1 mJ, wherein the step change depends on the size and number of formed trabecular bubbles. In this way autonomous functioning of laser pulse energy setting is enabled based on the algorithm 11 for bubble recognition and the algorithm 12 for laser pulse energy adjustment.

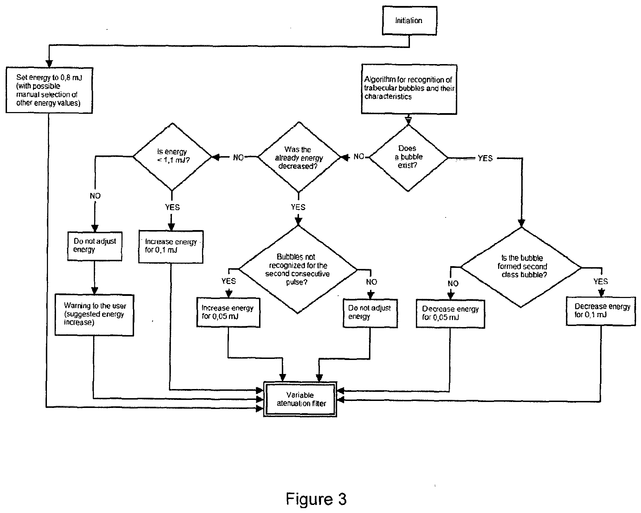

An embodiment example of the algorithm 12 for adjustment of energy in autonomous functioning of the therapeutic laser device for selective laser trabeculoplasty is shown on figure 3. During initialization the algorithm 12 sets the starting laser pulse energy at 0.8 mJ. The doctor can, based on his subjective judgment, manually select other starting value of laser pulse energy. After each executed laser pulse the algorithm 11 for recognition of trabecular bubbles sends information about existence and characteristics of bubbles into the algorithm 12 for adjustment of laser pulse energy. In case bubbles exist, the algorithm 12 determines whether bubbles are first or second class bubbles. If bubbles are first class bubbles, the pulse energy is decreased for 0.05 mJ, while in case bubbles are second class bubbles the pulse energy is decreased for 0.1 mJ. The bubble class is determined based on bubble number, size, speed and direction. A first class bubble is when the algorithm 11 for recognition of trabecular bubbles recognizes only one bubble. On the other hand, a second class bubble is when the algorithm 11 recognizes several bubbles or when a larger bubble is formed. If there are no bubbles, the algorithm 11 checks whether pulse energy during individual operation procedure has already been decreased. If energy was already decreased, the algorithm adjusts it in case bubbles were not formed in the last two consecutive pulses. The energy of the second consecutive pulse is in this case increased for 0.05 mJ. If laser pulse energy during operation procedure was not decreased, algorithm first checks if the current energy is lower than 1.1 mJ. This limitation of the highest energy is set based on the fact that typical energies, when performing SLT operations, do not exceed 1.2 mJ. If pulse energy is lower than 1.1 mJ, the algorithm increases energy for 0.1 mJ for the next laser pulse.

Otherwise, the algorithm 12 does not autonomously adjust the energy, but only warns the doctor that the energy threshold has been reached and that a manual increase of laser pulse energy is suggested. Pulse energy is adjusted with the help of the variable attenuation filter 7.

The described algorithm enables that the doctor performs SLT procedure close to the threshold for trabecular bubble formation. In this way, the described procedure unburdens the doctor, increases the reproducibility of operation and improves treatment effectiveness.

Ophthalmic laser device may have two cameras for taking images, which allow presentation of a three-dimensional image of taken images, which serves as additional information for the algorithm 11 for image recognition, and allows optimization of the whole algorithm and consequently more precise control of the variable attenuation filter 7, which decreases or increases laser pulse energy.

The ophthalmic laser device with the imaging system, which through the beam splitter takes images from the optical axis of a biomicroscope, with the help of the pre-set algorithm, enables automatic detection of bubble formation during operation procedures in a patient's eye. The design of this device allows autonomous adjustment of energy values of laser pulses on the basis of taken images during operations in ophthalmology.

## Claims

1. An ophthalmic laser device for performing selective laser trabeculoplasty, which comprises:
- a laser source (9) providing laser pulses,
- a beam splitter (5) for directing a part of an image from an optical axis (10),
- a slit lamp (4) for illumination of trabecular tissue,
- a binocular (6),
**characterized in that**
- the device further comprises:
- an imaging system (2) integrated via the beam splitter (5) before the binocular (6),
- a processor system (3),
- an algorithm (11),
- a predefined algorithm (12), and
- a variable attenuation filter (7) for regulation of laser pulse energy,
wherein the ophthalmic laser device is adapted to enable automatic detection of trabecular bubbles formation based on images taken by the imaging system (2) and analysed by the processor system (3), and wherein the processor system (3) and the predefined algorithm (12) based on the received images from the system (2) and the algorithm (11), are adapted to cause the ophthalmic laser device to control the variable attenuation filter (7), which is adapted to decrease or increase laser pulse energy.

2. The ophthalmic laser device according to claim 1, being adapted to detect number, size, speed and direction of movement of trabecular bubbles, which serves as an input information for decreasing or increasing the laser pulse energy.

3. The ophthalmic laser device according to claim 1 and claim 2, **characterized in that,** in addition to autonomous adjustment of laser energy, the device also includes a mode of action in which the device warns the doctor about existing trabecular bubbles and their characteristics.

4. The ophthalmic laser device according to claims 1 to 3, **characterized in that** in addition to autonomous adjustment of laser energy the device also includes a mode of action, in which the device suggests to the doctor an appropriate output laser energy based on existing trabecular bubbles and their characteristics.

5. The ophthalmic laser device according to claims 1 to 4, **characterized in that** the integrated slit lamp (4) is adapted to enable short-term functioning or emitting pulses with typical duration of 100 ms, which is triggered together with the laser pulse for reaching of an optimal contrast between a trabecular bubble and the surrounding tissue in order to take high quality images.

6. The ophthalmic laser device according to claims 1 to 5, **characterized in that,** the integrated imaging system (2) includes two cameras for imaging, which allow presenting a three-dimensional image of taken images and allow additional information for the algorithm (11) for image recognition.

7. The ophthalmic laser device according to claims 1 to 6, **characterized in that**, the laser pulse energy can be adjusted autonomously by the device or manually by the user.

8. The ophthalmic laser device according to any of the preceding claims, **characterized in that** the processor system (3) is adapted to cause the ophthalmic laser device to execute the following steps:
- after triggering the laser pulse, the recognition of bubble formation is performed by an algorithm (11) based on images from an imaging system (2)
- the algorithm (11) determines shapes similar to bubbles, in case they exist and determines their number, size, direction and speed of movement;
- speed, shape and sufficient size are three criteria for deciding whether trabecular bubble exists;
- the algorithm (11) forwards information about bubbles to the predefined algorithm (12) for regulation of laser pulse energy, the output information being bubble existence, their size and number.

9. The ophthalmic laser device according to claim 8, **characterized in that**
- the algorithm (11) is adapted to analyse more images taken by the imaging system (2) and to search for differences between consecutive images, which have been transformed into 1-bit, black and white images;
- that the algorithm (11) is adapted to add up differences of 1-bit images and thereby to gain information about objects that have moved;
- that the algorithm (11) is then adapted to select at least two 1-bit images of movement and based on pre-set criteria to determine whether imaged shapes are bubbles;
- that the pre-set criteria are shape, speed and size of bubble (object);
- that in case a bubble is recognized, the algorithm (11) determines whether the bubble is small or large based on its size and speed.

10. The ophthalmic laser device according to claim 8 and claim 9, **characterized in that** the algorithm (11) for bubble recognition is adapted to forward information about bubble existence and their characteristics to the predefined algorithm (12), which based on predefined values with the help of a variable attenuation filter (7) appropriately decreases or increases laser pulse energy.

11. The ophthalmic laser device according to claim 10, **characterized in that** the processor system (3) is adapted to cause the ophthalmic laser device to execute the following steps:
- after each executed laser pulse the algorithm (11) sends information existence and characteristics of bubbles to the algorithm (12) for adjustment of laser pulse energy;
- that in case bubbles exist, the algorithm (12) determines whether bubbles are first or second class bubbles based on bubble number, size, speed and direction;
- that for first class bubbles, which means that only one bubble is present, the pulse energy is decreased for 0.05 mJ,
- that for second class bubbles, which means that more bubbles are present, the pulse energy is decreased for 0.1 mJ;
- that in case of no bubbles, the algorithm (11) checks whether pulse energy during individual operation procedure has already been decreased;
- that in case energy was already decreased, the algorithm adjusts it in case bubbles were not formed in the last two consecutive pulses and the energy of the second consecutive pulse is in this case increased for 0.05 mJ;
- that in case the laser pulse energy during operation procedure has not been decreased, the algorithm (12) first checks if the current energy is lower than 1.1 mJ;
- that in case the pulse energy is lower than 1.1 mJ, the algorithm increases energy for 0.1 mJ for the next laser pulse; and
- in case the current energy is 1.1 mJ, the algorithm (12) does not autonomously adjust the energy, but only warns the doctor that the energy threshold has been reached and that a manual increase of laser pulse energy is suggested.

## Patentansprüche

1. Augenlaservorrichtung zur Durchführung einer selektiven Laser-Trabekuloplastik umfassend:
- eine Laser-Quelle (9), die Laserpulse bereitstellt,
- einen Strahlteiler (5) zur Leitung eines Bildteils von der optischen Achse (10),
- eine Spaltlampe (4) zur Beleuchtung des trabekulären Gewebes,
- ein Binokular (6),
**dadurch gekennzeichnet, dass**
- die Vorrichtung des Weiteren umfasst:
- ein Bildgebungssystem (2) das über den Strahlteiler (5) vor dem Binokular (6) integriert ist,
- ein Prozessorsystem (3),
- einen Algorithmus (11),
- einen vorbestimmten Algorithmus (12) sowie
- einen variablen Dämpfungsfilter (7) zur Steuerung der Laserpulsenergie,
wobei die Augenlaservorrichtung eingerichtet ist, um aufgrund der Bilder, die vom Bildgebungssystem (2) erfasst und vom Prozessorsystem (3) analysiert wurden, eine automatische Erkennung der Bildung von Trabekelblasen zu ermöglichen, und
wobei das Prozessorsystem (3) und der vorbestimmte Algorithmus (12) aufgrund der vom System (2) und dem Algorithmus (11) empfangenen Bilder eingerichtet sind, um zu bewirken, dass die Augenlaservorrichtung den variablen Dämpfungsfilter (7) steuert, der eingerichtet ist, um die Laserpulsenergie zu verringern oder zu erhöhen.

2. Augenlaservorrichtung nach Anspruch 1, die eingerichtet ist, um die Anzahl, die Größe, die Geschwindigkeit und die Bewegungsrichtung der Trabekelblasen zu erkennen, was als Eingangsinformationen zur Verringerung oder Erhöhung der Laserpulsenergie dient.

3. Augenlaservorrichtung nach Anspruch 1 oder Anspruch 2, **dadurch gekennzeichnet, dass** zusätzlich zur autonomen Einstellung der Laserenergie die Vorrichtung auch eine Wirkungsweise einschließt, bei der die Vorrichtung den Arzt vor bestehenden Trabekelblasen und deren Eigenschaften warnt.

4. Augenlaservorrichtung nach Ansprüchen 1 bis 3, **dadurch gekennzeichnet, dass** zusätzlich zur autonomen Einstellung der Laserenergie die Vorrichtung auch eine Wirkungsweise einschließt, bei der die Vorrichtung dem Arzt eine geeignete Ausgangslaserenergie wegen bestehender Trabekelblasen und deren Eigenschaften vorschlägt.

5. Augenlaservorrichtung nach Ansprüchen 1 bis 4, **dadurch gekennzeichnet, dass** die integrierte Spaltlampe (4) eingerichtet ist, um eine Kurzzeitfunktion zu ermöglichen oder Pulse mit einer typischen Dauer von 100 ms abzugeben, was zusammen mit dem Laserpuls ausgelöst wird, um einen optimalen Kontrast zwischen einer Trabekelblase und dem benachbarten Gewebe zu erreichen, um Hochqualitätsbilder zu erfassen.

6. Augenlaservorrichtung nach Ansprüchen 1 bis 5, **dadurch gekennzeichnet, dass** das integrierte Bildgebungssystem (2) zwei Bildgebungskameras umfasst, die eine Darstellung eines dreidimensionalen Bildes der erfassten Bilder und zusätzliche Informationen für den Algorithmus (11) zur Bilderkennung ermöglichen.

7. Augenlaservorrichtung nach Ansprüchen 1 bis 6, **dadurch gekennzeichnet, dass** die Laserpulsenergie autonom durch die Vorrichtung oder manuell vom Benutzer einstellbar ist.

8. Augenlaservorrichtung nach einem der vorhergehenden Ansprüche, **dadurch gekennzeichnet, dass** das Prozessorsystem (3) eingerichtet ist, um zu bewirken, dass die Augenlaservorrichtung die folgenden Schritte ausführt:
- nach Laserpulsaktivierung wird die Erkennung der Blasenbildung durch einen Algorithmus (11) aufgrund der Bilder vom Bildgebungssystem (2) ausgeführt;
- der Algorithmus (11) erfasst blasenähnliche Formen, ob sie existieren, und bestimmt deren Anzahl, Größe, Richtung und Bewegungsgeschwindigkeit;
- drei Kriterien zur Entscheidung, ob eine Trabekelblase vorliegt, sind Geschwindigkeit, Form und eine ausreichende Größe;
- der Algorithmus (11) sendet die Information hinsichtlich der Blasen zum vorbestimmten Algorithmus (12) zur Steuerung der Laserpulsenergie, wobei die Ausgangsinformation, das Vorhandensein der Blasen, deren Größe und ihre Anzahl ist.

9. Augenlaservorrichtung nach Anspruch 8, **dadurch gekennzeichnet, dass**
- der Algorithmus (11) eingerichtet ist, um mehrere vom Bildgebungssystem (2) erfassten Bilder zu analysieren und die Unterschiede zwischen den aufeinanderfolgenden Bildern, die in 1-bit, schwarz-weiß Bilder umwandelt wurden, zu suchen;
- dass der Algorithmus (11) eingerichtet ist, um die Unterschiede der 1-bit Bilder zu addieren und hierdurch Informationen über die Objekte, die bewegt wurden, zu gewinnen;
- dass der Algorithmus (11) weiterhin eingerichtet ist, um mindestens zwei 1-bit Bilder der Bewegung auszuwählen und aufgrund der vorbestimmten Kriterien festzustellen, ob die abgebildeten Formen Blasen sind;
- dass die vorbestimmten Kriterien Form, Geschwindigkeit und Größe der Blase (Objekt) sind;
- dass, falls eine Blase erkannt wurde, der Algorithmus (11) auf Basis ihrer Größe und Geschwindigkeit bestimmt, ob die Blase klein oder groß ist.

10. Augenlaservorrichtung nach Anspruch 8 oder Anspruch 9, **dadurch gekennzeichnet, dass** der Algorithmus (11) zur Blasenerfassung eingerichtet ist, um die Information über das Vorhandsein der Blase und ihre Eigenschaften an einen vorbestimmten Algorithmus (12) weiterzuleiten, der auf Grund der vorbestimmten Werte, mit Hilfe des variablen Dämpfungsfilters (7) die Laserpulsenergie entsprechend verringert oder erhöht.

11. Augenlaservorrichtung nach Anspruch 10, **dadurch gekennzeichnet, dass** das Prozessorsystem (3) eingerichtet ist, um zu bewirken, dass die Augenlaservorrichtung, die folgenden Schritte ausführt:
- nach jedem ausgeführten Laserpuls sendet der Algorithmus (11) die Information über das Vorhandsein und die Eigenschaften der Blasen an den Algorithmus (12) zur Einstellung der Laserpulsenergie;
- dass im Falle, dass Blasen vorhanden sind, der Algorithmus (12) aufgrund ihrer Anzahl, Größe, Geschwindigkeit und Richtung feststellt, ob es sich um Blasen ersten oder zweiten Grades handelt
- dass für die Blasen ersten Grades, was bedeutet, dass nur eine Blase vorhanden ist, die Pulsenergie um 0,05 mJ verringert wird;
- dass für Blasen zweiten Grades, was bedeutet, dass mehrere Blasen vorhanden sind, die Pulsenergie um 0.1 mJ verringert wird;
- dass im Falle des Nichtvorhandenseins von Blasen der Algorithmus (11) überprüft, ob die Pulsenergie während des Einzeloperationsvorgangs bereits verringert wurde;
- dass der Algorithmus im Falle, dass die Energie bereits verringert wurde, sie einstellt, im Falle, dass sich in den letzten beiden aufeinanderfolgenden Pulsen keine Blasen gebildet haben und die Energie des zweiten folgenden Pulses in diesem Falle um 0.05 mJ erhöht wird;
- dass im Falle, dass die Laserpulsenergie während des Operationsvorgangs nicht verringert wurde, der Algorithmus (12) zuerst überprüft, ob die aktuelle Energie niedriger als 1,1 mJ ist;
- dass im Falle, dass die aktuelle Energie niedriger als 1,1 mJ ist, der Algorithmus die Energie um 0,1 mJ für die nächsten Laserpulse erhöht; und
- im Falle, dass die aktuelle Energie 1,1 mJ beträgt, der Algorithmus (12) die Energie nicht autonom einstellt, sondern den Arzt warnt, dass die Energieschwelle erreicht wurde und dass eine manuelle Erhöhung der Laserpulsenergie vorgeschlagen wird.

## Revendications

1. Un dispositif laser ophtalmique pour effectuer une trabéculoplastie sélective au laser comprenant :
- une source laser (9) fournissant des impulsions laser,
- un diviseur de faisceau (5) permettant de diriger une partie d'une image à partir d'un axe optique (10),
- une lampe à fente (4) pour l'illumination d'un tissu trabéculaire,
- un binoculaire (6),
**caractérisé en ce que**
- le dispositif comprend en outre :
- un système d'imagerie (2) intégré par l'intermédiaire du diviseur de faisceau (5) avant le binoculaire (6),
- un système de processeur (3),
- un algorithme (11),
- un algorithme prédéfini (12) et
- un filtre atténuateur variable (7) pour le contrôle de l'énergie des impulsions laser,
le dispositif laser ophtalmique étant conçu pour permettre une détection automatique de formation de bulles trabéculaires sur la base d'images prises par le système d'imagerie (2) et analysées par le système de processeur (3) et
le système de processeur (3) et l'algorithme prédéfini (12), en fonction des images reçues du système (2) et de l'algorithme (11), étant conçus pour amener le dispositif laser ophtalmique à commander le filtre d'atténuation variable (7) qui est adapté de manière à diminuer ou augmenter l'énergie des impulsions laser.

2. Le dispositif laser ophtalmique selon la revendication 1, qui est adapté pour détecter le nombre, la taille, la vitesse et la direction du mouvement des bulles trabéculaires, ce qui sert d'informations d'entrée pour diminuer ou augmenter l'énergie des impulsions laser.

3. Le dispositif laser ophtalmique selon la revendication 1 ou la revendication 2, **caractérisé en ce que,** outre le réglage autonome de l'énergie laser, le dispositif comprend également un mode d'action dans lequel le dispositif avertit le médecin de la présence de bulles trabéculaires et de leurs caractéristiques.

4. Le dispositif laser ophtalmique selon les revendications 1 à 3, **caractérisé en ce que,** outre le réglage autonome de l'énergie laser, le dispositif comprend également un mode d'action dans lequel le dispositif suggère au médecin une énergie laser de sortie appropriée en fonction des bulles trabéculaires existantes et leurs caractéristiques.

5. Le dispositif laser ophtalmique selon les revendications 1 à 4, **caractérisé en ce que** la lampe à fente intégrée (4) est conçue pour permettre un fonctionnement à court terme ou émettre des impulsions avec une durée typique de 100 ms, qui est déclenché conjointement avec l'impulsion laser pour atteindre un contraste optimal entre une bulle trabéculaire et le tissu environnant afin de prendre des images de haute qualité.

6. Le dispositif laser ophtalmique selon les revendications 1 à 5, **caractérisé en ce que** le système d'imagerie intégré (2) comprend deux caméras pour l'imagerie, qui permettent de présenter une image tridimensionnelle d'images prises et d'apporter des informations supplémentaires pour l'algorithme (11) en vue de la reconnaissance d'image.

7. Le dispositif laser ophtalmique selon les revendications 1 à 6, **caractérisé en ce que** l'énergie d'impulsions laser peut être ajustée de manière autonome par le dispositif ou manuellement par l'utilisateur.

8. Le dispositif laser ophtalmique selon l'une quelconque des revendications précédentes, **caractérisé en ce que** le système de processeur (3) est adapté pour amener le dispositif laser ophtalmique à exécuter les opérations suivantes :
- après l'activation des impulsions laser, la reconnaissance de la formation de bulles est effectuée par un algorithme (11) à partir d'images d'un système d'imagerie (2) ;
- l'algorithme (11) détermine les formes similaires aux bulles, si elles existent, et détermine leur nombre, leur taille, leur direction et leur vitesse de déplacement ;
- la vitesse, la forme et la taille suffisante sont les trois critères retenus pour décider si la bulle trabéculaire existe ;
- l'algorithme (11) transmet des informations concernant des bulles à l'algorithme prédéfini (12) pour réguler l'énergie des impulsions laser, les informations de sortie étant l'existence de bulles, leur taille et leur nombre.

9. Le dispositif laser ophtalmique selon la revendication 8, **caractérisé en ce que**
- l'algorithme (11) est conçu pour analyser plus d'images prises par le système d'imagerie (2) et pour rechercher les différences entre les images consécutives, qui ont été transformées en images 1 bit en noir et blanc ;
- l'algorithme (11) est conçu pour ajouter des différences d'images à 1 bit et obtient ainsi des informations sur les objets qui se sont déplacés ;
- l'algorithme (11) est ensuite adapté pour choisir au moins deux images de mouvement 1 bit et, sur la base des critères préétablis, pour déterminer si les formes observées par imagerie sont des bulles ;
- les critères préétablis sont la forme, la vitesse et la taille de la bulle (objet) ;
- dans le cas où une bulle est reconnue, l'algorithme (11) détermine si la bulle est petite ou grande en fonction de sa taille et de sa vitesse.

10. Le dispositif laser ophtalmique selon la revendication 8 et la revendication 9, **caractérisé en ce que** l'algorithme (11) pour la reconnaissance de la bulle est conçu pour transmettre des informations sur l'existence de la bulle et leurs caractéristiques à l'algorithme prédéfini (12), qui, en fonction des valeurs prédéfinies à l'aide d'un filtre d'atténuation variable (7) diminue ou augmente l'énergie des impulsions laser de manière appropriée.

11. Le dispositif laser ophtalmique selon la revendication 10, **caractérisé en ce que** le système de processeur (3) est adapté pour amener le dispositif laser ophtalmique à exécuter les opérations suivantes :
- après chaque impulsion laser exécutée, l'algorithme (11) envoie des informations sur l'existence et les caractéristiques des bulles à l'algorithme (12) pour le réglage de l'énergie des impulsions laser ;
- dans le cas où des bulles existent, l'algorithme (12) détermine si les bulles sont des bulles de première ou de seconde classe sur la base du nombre de bulles, de leur taille, de leur vitesse et de leur direction ;
- pour les bulles de première classe, ce qui signifie que seule une bulle est présente, l'énergie d'impulsion est diminuée de 0,05 mJ ;
- pour les bulles de seconde classe, ce qui signifie que plusieurs bulles sont présentes, l'énergie d'impulsion est diminuée de 0,1 mJ ;
- en cas d'absence de bulles, l'algorithme (12) vérifie si l'énergie des impulsions pendant la procédure d'opération individuelle a déjà été réduite ;
- si l'énergie a déjà été réduite, l'algorithme l'ajuste dans le cas où aucune bulle n'a pas été formée pendant les deux dernières impulsions consécutives et dans ce cas, l'énergie de la deuxième impulsion consécutive est augmentée de 0,05 mJ ;
- dans le cas où l'énergie d'impulsions laser pendant la procédure d'opération n'a pas été réduite, l'algorithme (12) vérifie d'abord si l'énergie actuelle est inférieure à 1,1 mJ;
- dans le cas où l'énergie d'impulsion est inférieure à 1,1 mJ, l'algorithme augmente l'énergie de 0,1 mJ pour l'impulsion laser suivante ; et
- dans le cas où l'énergie actuelle est de 1,1 mJ, l'algorithme (12) n'ajuste pas l'énergie de façon autonome, mais signale au médecin que le seuil d'énergie a été atteint et suggère une augmentation manuelle de l'énergie d'impulsions laser.
